# EUROPÄISCHE PATENTSCHRIFT

(11) **EP 0 815 796 B1**
(45) Veröffentlichungstag und Bekanntmachung des Hinweises auf die Patenterteilung: **04.06.2003**
(21) Anmeldenummer: 97110858.4
(22) Anmeldetag: 01.07.1997
(51) Int. Cl.: A61B 17/34, A61B 18/12

(54) **Trokar für laparoskopische Operationen**
Trocar for laparoscopic operations
Trocart pour opérations laparoscopiques

(30) Priorität: 01.07.1996 DE 19626408
(43) Veröffentlichungstag der Anmeldung: 07.01.1998
(73) Patentinhaber: Berchtold Holding GmbH, 78532 Tuttlingen (DE)
(72) Erfinder: Hofmann, Helge, 78576 Emmingen (DE); Fritzsch, Gernod, 78532 Tuttlingen (DE); Hill, Wolfram, 79102 Freiburg (DE)
(74) Vertreter: Manitz, Gerhart, Dr.

(56) Entgegenhaltungen:
- EP-A- 0 547 772
- WO-A-93/13718
- WO-A-94/25110
- DE-A- 4 116 648
- US-A- 5 445 142

## Beschreibung

Die Erfindung betrifft ein Trokar für laparoskopische Operationen von Patienten nach dem Oberbegriff des Patentanspruchs 1.

Bei einem bekannten Trokar dieser Art (DE 93 90 139 U1) sind die Hochfrequenzelektroden am vorderen Ende des Rohres angeordnet und können ggf. nach Einbringen des Rohres in den Körper des Patienten herausgenommen werden. Abgesehen davon, daß das Einführen und Herausnehmen der Elektroden eine erhebliche Geschicklichkeit des Operateurs verlangt, hängt die Wirksamkeit des Schneidvorganges bei diesem bekannten Trokar von der Stromleitfähigkeit des beaufschlagten Gewebes ab. Dadurch ist der Schneideffekt während des Einstechens des Trokars stark veränderlich und vom Operateur kaum beherrschbar.

Weiter ist es bereits bekannt (US-A-53 80 291), in ein Trokar ein elektronisches Endoskop mit einer mechanischen, transparenten Spitze einzubringen, welche beim Einführen des Trokars in den Körper den Einstechkanal bildet. Bei diesem Instrument erfolgt das Einstechen des Trokars auf rein mechanischem Wege, was die Gefahr von Blutungen mit sich bringt.

Aus der US-A-5 445 142 ist bereits ein Trokar für laporoskopische Operationen vom Patienten mit einem Rohr bekannt, in welchem ein rohrförmiger Schneidstab axial verschiebbar und in einer Schneidposition feststellbar angebracht ist. Am vorderen Ende des Schneidstabes sind an einen Hochfrequenzgenerator anschließbare Hochfrequenzanschlüsse und an diese angeschlossene, durch den Hochfrequenzstrom aufheizbare elektrochirurgische Schneidmittel zum elektrochirurgischen Schneiden von Gewebe beim Eindringen des Rohres in den Körper des Patienten angeordnet. Die Schneidmittel befinden sich in der Schneidposition im Bereich des vorderen Ende des Rohres, wobei in dem Schneidstab ein Endoskop mit seinem vorderen Beobachtungsende unmittelbar hinter den Schneidmitteln untergebracht ist. Das vordere Ende des rohrförmigen Schneidstabes ist durch ein transparentes Bauelement verschlossen.

Die EP 0 547 772 A1 beschreibt ein laparoskopisches Instrument, in dessen Rohr ein Fluidkanal vorgesehen ist.

Das Ziel der Erfindung besteht darin, ein Trokar der eingangs genannten Gattung zu schaffen, bei dem einer durch Verunreinigungen bedingten verschlechterten Sicht durch das Endoskop entgegengewirkt wird.

Zur Lösung dieser Aufgabe sind die Merkmale des kennzeichnenden Teils des Patentanspruchs 1 vorgesehen.

Auf diese Weise wird Verunreinigungen des Endoskops bzw. des transparenten Bauelements entgegengewirkt. Hierfür eignet sich insbesondere das bei laporoskopischen Operationen zum Füllen des Bauchraumes ohnehin erforderliche Kohlendioxid. In gleicher Weise könnte jedoch mit einer Spüllösung gearbeitet werden.

Aufgrund der Ausführungsform nach Anspruch 2 wird eine elektrochirurgische Schneidwirkung über den gesamten Querschnitt des Trokars erzielt, und zwar vor allem dann, wenn in bevorzugter Weise der Durchmesser des Schneidstabes nur wenig geringer als der Innendurchmesser des Trokar-Rohres ist und die elektrochirurgischen Schneidmittel sich nicht nur innerhalb des Querschnitts des Schneidstabes, sondern auch möglichst weit bis zu dessen Rand erstrecken.

Besonders bevorzugt sind die Schneidmittel nach Anspruch 3 oder 4 ausgebildet. Drähte, Klingen oder Messer besitzen eine gute elektrochirurgische Schneidwirkung und ermöglichen es außerdem, dass um sie herum noch ausreichend Freiraum für eine freie Sicht zum Operationsfeld mittels eines im Bereich des vorderen Endes des Schneidstabes bevorzugt vorgesehenen Endoskopes vorhanden sind.

Das Einstechen des Trokars wird weiter durch die Maßnahmen nach Anspruch 5 begünstigt.

Eine besonders gleichmäßige Schneidwirkung wird durch die Merkmale des Anspruches 6 erzielt.

Die Weiterbildung nach Anspruch 7 gewährleistet eine gleichmäßige Schneidwirkung über den gesamten Stabquerschnitt bei gleichzeitig nur geringfügig durch die Schneidmittel behinderter Sicht zur Schneidstelle.

Eine bevorzugte Ausbildung des transparenten Bauelements entnimmt man Anspruch 8.

Die Ausführungsformen nach den Ansprüchen 9 oder 10 hat den Vorteil, dass das den rohrförmigen Schneidstab vorne verschließende transparente Bauelement selbst durch die erhitzten Schneidmittel nur wenig erwärmt wird.

Die elektrochirurgischen Schneidmittel werden bevorzugt durch Hochfrequenz-Stromimpulse gespeist, wodurch beim Einstechen das Gewebe unter Sicht schichtweise geschnitten werden kann.

Als Draht für die Schneidmittel eignet sich z.B. Wolframdraht mit einem Durchmesser von 0,2 mm.

Die Erfindung wird im folgenden beispielsweise anhand der Zeichnung beschrieben; in dieser zeigt:
- Figur 1: eine vergrößerte schematische Querschnittsansicht des vorderen Endes eines erfindungsgemäßen Trokars, wobei außerdem das vordere Ende eines eingeführten Endoskops und rein schematisch der Anschluß an einen Hochfrequenz-Generator sowie eine Spülgasversorgung wiedergegeben sind,
- Figur 2: eine zu Figur 1 analoge Schnittansicht einer weiteren Ausführungsform und die
- Figuren 3a, 3b und 3c: Stirnansichten des rohrförmigen Schneidstabes nach den Figuren 1 oder 2 mit verschiedenen Anordnungen der elektrochirurgischen Schneidmittel.

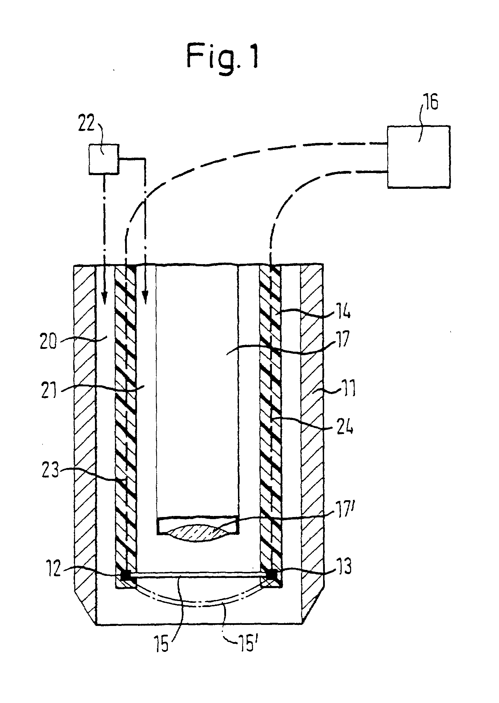

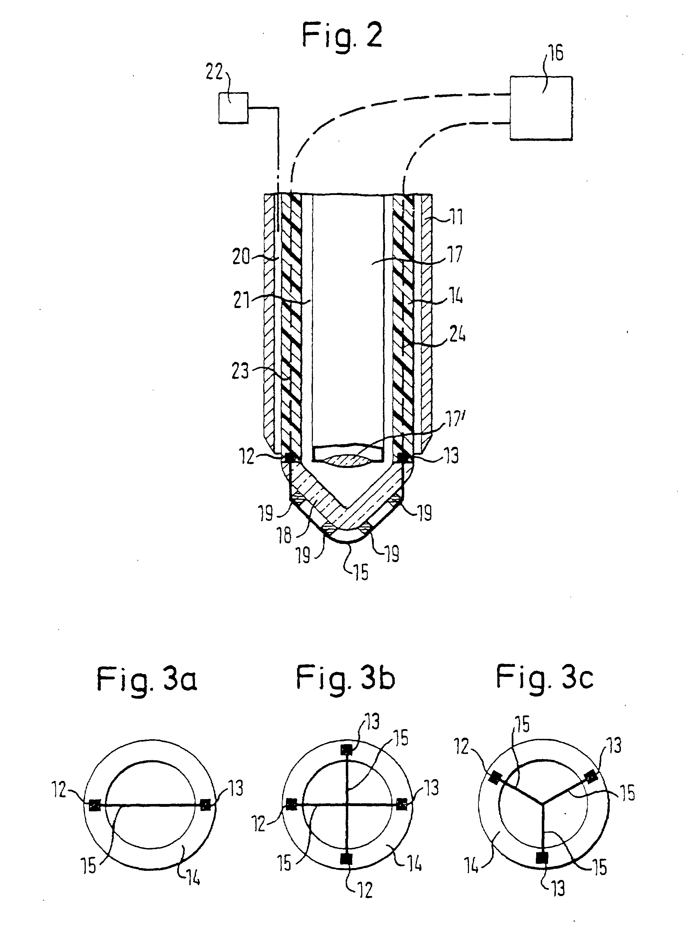

Nach Figur 1 ist im vorne angeschärften Rohr 11 eines erfindungsgemäßen Trokars ein rohrförmiger Schneidstab 14 axial verschiebbar so angeordnet, daß sein vorderes Ende mit dem vorderen Ende des Rohres 11 etwa bündig ist, nach Figur 1 etwas gegenüber dem vorderen Ende des Rohres 11 zurückversetzt ist oder in bevorzugter Weise geringfügig über das vordere Ende des Rohres 11 vorsteht. Der rohrförmige Schneidstab 14 besteht vorzugsweise aus Kunststoff und enthält zwei an einen Hochfrequenzgenerator 16 angeschlossene Hochfrequenzenergie- Zuleitungen 23 bzw. 24, die im Bereich des vorderen Endes des Schneidstabes 14 jeweils einen Anschluß 12 bzw. 13 aufweisen, zwischen denen sich ein Schneiddraht 15 oder alternativ eine Schneidklinge 15' erstreckt. Während der Schneiddraht 15 den freien Querschnitt am vorderen Ende des Schneidstabes 14 geradlinig und diametral überspannt (Figur 3a), steht die Schneidklinge 15' bogenförmig vor das vordere Ende des rohrförmigen Schneidstabes 14 vor.

Im Innenraum 21 des rohrförmigen Schneidstabes 14 befindet sich ein Endoskop 17, welches soweit eingeschoben ist, daß sein Beobachtungsende 17' sich nahe der vorderen Öffnung des rohrförmigen Schneidstabes 14 bzw. des Rohres 11 befindet.

Ein zwischen dem Rohr 11 und dem rohrförmigen Schneidstab 14 belassener Zwischenraum 20 und/oder der Innenraum 21 des rohrförmigen Schneidstabes 14 ist bzw. sind an eine außerhalb des Trokars vorgesehene Spülgas- oder Spülflüssigkeitsversorgung 22 angeschlossen.

Bei der Ausführungsform nach Figur 2, in der gleiche Bezugszahlen die gleichen Bauelemente wie in Figur 1 bezeichnen, ist das vordere Ende des vorförmigen Schneidstabes 14 durch eine transparente Kuppel 18 verschlossen, wobei der Schneiddraht 15 in einem Abstand vor der Kuppel 18 verläuft und durch Abstandshalter 19 in einem definierten Abstand von der Außenfläche der Kuppel 18 gehalten wird.

### Die Funktion des beschriebenen Trokars ist wie folgt:

Vor dem Einstich in den Körper eines Patienten wird der rohrförmige Schneidstab 14 in eine der aus Figur 1 und 2 ersichtlichen Positionen relativ zum Trokar-Rohr 11 gebracht, so daß die elektrochirurgischen Schneidmittel 15, 15' sich im Bereich des vorderen Endes des Trokar-Rohres 11 befinden.

Anschließend wird das Endoskop 17 in das Innere des rohrförmigen Schneidstabes 14 eingebracht, und zwar so nah wie möglich an die elektrochirurgischen Schneidmittel 15, 15' (Figur 1) bzw. die transparente Kuppel 18 (Figur 2). Nunmehr wird vom Hochfrequenzgenerator 16 Hochfrequenzenergie an die elektronischen Schneidmittel 15, 15' angelegt, so daß diese eine für den Schneidvorgang erforderlicher Temperatur annehmen. Nunmehr kann das Trokar auf den Körper aufgesetzt und in diesen unter entscheidender Mitwirkung der elektrochirurgischen Schneidmittel 15, 15' in das Gewebe des Körpers eingestochen werden, wobei der Einstechvorgang durch das Endoskop 17 vom Operateur genau beobachtet werden kann. Hierdurch kann ein Verletzungsrisiko von empfindlichen Blutgefäßen oder bestimmter innerer Organe wie des Darmes ganz erheblich reduziert werden. Außerdem wird durch den elektrochirurgischen Schnittvorgang gewährleistet, daß evtl. angeschnittene Blutgefäße koaguliert und damit Blutungen gestillt werden. Auch der Kraftaufwand für das Einstechen des erfindungsgemäßen Trokars ist gegenüber bekannten Trokaren deutlich herabgesetzt, so daß der Einstechvorgang auch wesentlich empfindlicher und mit mehr Gefühl vorgenommen werden kann.

Nachdem das Trokar-Rohr 11 die gewünschte Position im Körper erreicht hat, werden das Endoskop 17 und der rohrförmige Schneidstab 14 aus dem Rohr 11 herausgenommen, worauf dann entweder das Endoskop 17 allein erneut eingeschoben wird, um den anschließenden Operationsvorgang zu beobachten, oder ein geeignetes medizinisches Instrument in das Rohr eingebracht wird.

Bei der Ausführungsform nach Figur 1 wird von der Versorgung 22 ein Spülgas oder eine Spülflüssigkeit bevorzugt in den Innenraum 21 des rohrförmigen Schneidstabs 14 eingebracht, weil dann der Raum zwischen dem Beobachtungsende 17' des Endoskops 17 und den elektrochirurgischen Schneidmitteln 15, 15' laufend wirksam gespült und damit eine Verunreinigung des Beobachtungsendes 17' des Endoskops 17 vermieden wird. Zusätzlich kann die Versorgung 22 auch noch an einen zwischen dem Rohr 11 und dem Schneidstab 14 vorgesehenen Zwischenraum 20 angelegt werden, um in an sich bekannter Weise einen Spülung des Operationsfeldes herbeizuführen.

Beim Ausführungsbeispiel nach Figur 2 sollte dagegen die Versorgung 22 an den Zwischenraum 20 zwischen dem Rohr 11 und dem Schneidstab 14 angelegt werden, um die Außenfläche der Kuppel 18 so zu spülen, daß sich dort beim Schneidvorgang entstehende Verunreinigungen nicht festsetzen können.

Die technisch einfachste Lösung für die Anordnung eines Schneiddrahtes 15 entnimmt man Figur 3a. Hier wird einfach ein geradliniger Schneiddraht 15 diametral über das vordere Ende des Innenquerschnitt des rohrförmigen Schneidstabes 14 gespannt und an den diametral gegenüberliegenden Anschlüssen 12, 13 in geeigneter Weise befestigt.

Bei der kreuzförmigen Anordnung zweier Schneiddrähte 15 gemäß Figur 3b ist eine gleichmäßigere Schneidwirkung über den Querschnitt des Schneidstabes 14 gewährleistet. In diesem Fall müssen jedoch zwei Anschlußpaare 12, 13 winkelmaßig um 90° gegeneinander versetzt vorgesehen werden.

Weiter ist eine dreizackige Sternanordnung des Schneiddrahtes 15 gemäß Figur 3c denkbar, wozu drei Anschlüsse 12, 13, 13 um einen Winkel von 120° gegeneinander versetzt an der Stirnseite des rohrförmigen Schneidstabes 14 anzuordnen sind.

### Bezugszeichenliste

- 11: Rohr
- 12: Anschluß
- 13: Anschluß
- 14: Schneidstab
- 15: Schneiddraht
- 15': Schneidklinge
- 16: Hochfrequenzgenerator
- 17: Endoskop
- 17': Beobachtungsende
- 18: transparentes Bauelement (Kuppel)
- 19: Abstandshalter
- 20: Zwischenraum
- 21: Innenraum
- 22: Spülgas-, Spülflüssigkeitsversorgung
- 23: Hochfrequenzenergie-Zuleitung
- 24: Hochfrequenzenergie-Zuleitung

## Patentansprüche

1. Trokar für laparoskopische Operationen von Patienten mit einem Rohr (11), in dem ein rohrförmiger Schneidstab (14) axial verschiebbar und in einer Schneidposition feststellbar angebracht ist, an dessen vorderem Ende an einen Hochfrequenzgenerator (16) anschließbare Hochfrequenz-Anschlüsse (12, 13) und an diese angeschlossene, durch den Hochfrequenzstrom aufheizbare elektrochirurgische Schneidmittel (15, 15') zum elektrochirurgischen Schneiden von Gewebe beim Eindringen des Rohres (11) in den Körper des Patienten angeordnet sind, die sich in der Schneidposition im Bereich des vorderen Endes des Rohres (11) befinden, wobei in dem Schneidstab (14) ein Endoskop (17) mit seinem vorderen Beobachtungsende (17') unmittelbar hinter den Schneidmitteln (15, 15') untergebracht ist,
**dadurch gekennzeichnet,**
**dass** zwischen der Innenwand des rohrförmigen Schneidstabes (14) und der Außenwand des Endoskops (17) ein einen Teil des Innenraums (21) des das Endoskop (17) aufnehmenden, rohrförmigen Schneidstabes (14) bildender, an eine Spülgas- oder Spülflüssigkeitsversorgung (22) anschließbarer Zwischenraum und/oder, sofern das vordere Ende des rohrförmigen Schneidstabes (14) durch ein transparentes Bauelement (18) verschlossen ist, zwischen der Innenwand des Rohres (11) und der Außenwand des rohrförmigen Schneidstabes (14) ein an eine Spülgas- oder Spülflüssigkeitsversorgung (22) anschließbarer Zwischenraum (20) vorgesehen ist.

2. Trokar nach Anspruch 1,
**dadurch gekennzeichnet,**
**dass** die Schneidmittel (15, 15') das vordere Ende des Schneidstabes (14) überspannen.

3. Trokar nach Anspruch 2,
**dadurch gekennzeichnet,**
**dass** die Schneidmittel (15, 15') länglich ausgebildet sind.

4. Trokar nach Anspruch 3,
**dadurch gekennzeichnet,**
**dass** die Schneidmittel an die Anschlüsse (12, 13) angeschlossene Drähte (15), Klingen oder Messer (15') sind.

5. Trokar nach einem der vorhergehenden Ansprüche,
**dadurch gekennzeichnet,**
**dass** die Schneidmittel (15, 15') bogenförmig oder spitzenartig vom vorderen Ende des Schneidstabes (14) vorstehen.

6. Trokar nach einem der Ansprüche 3 bis 5,
**dadurch gekennzeichnet,**
**dass** die Schneidmittel (15, 15') den vorderen Querschnitt des Schneidstabes (14) diametral oder kreuz- oder sternförmig überspannen.

7. Trokar nach Anspruch 6,
**dadurch gekennzeichnet,**
**dass** die Schneidmittel (15, 15') den freien Innenquerschnitt des rohrförmigen Schneidstabes (14) überspannen.

8. Trokar nach einem der vorhergehenden Ansprüche,
**dadurch gekennzeichnet,**
**dass** das transparente Bauelement (18) kuppelartig ausgebildet ist.

9. Trokar nach einem der vorhergehenden Ansprüche,
**dadurch gekennzeichnet,**
**dass** die Schneidmittel (15, 15') einen Abstand vom transparenten Bauelement (18) aufweisen.

10. Trokar nach Anspruch 9,
**dadurch gekennzeichnet,**
**dass** der Abstand durch Abstandshalter (19) aufrecht erhalten ist.

## Claims

1. Trocar for laparoscopic operations on patients comprising a tube (11) in which a tubular cutting bar (14) is fitted, the tubular cutting bar (14) being axially displaceable and fixable in a cutting position, wherein high frequency connections (12, 13) connectable to a high frequency generator (16) and electrosurgical cutting means (15, 15') connected thereto and heatable by the high frequency current are arranged at the front end of the tubular cutting bar (14) for the electrosurgical cutting of tissue on penetration of the tube (11) into the patient's body, the electrosurgical cutting mean being located in the cutting position in the region of the front end of the tube (11) and wherein an endoscope (17) is accommodated in the cutting bar (14) with its front observation end (17') directly behind the cutting means (15, 15'),
**characterised in that**
an intermediate space is present between the inner wall of the tubular cutting bar (14) and the outer wall of the endoscope (17) which forms part of the inner space (21) of the tubular cutting bar (14) receiving the endoscope (17) and is connectable to a flushing gas or flushing liquid supply (22) and/or, provided the front end of the tubular cutting bar (14) is closed by a transparent component (18), an intermediate space (20) is provided between the inner wall of the tube (11) and the outer wall of the tubular cutting bar (14) and can be connected to a flushing gas or flushing liquid supply (22).

2. Trocar in accordance with claim 1, **characterised in that** the cutting means (15, 15') span the front end of the cutting bar (14).

3. Trocar in accordance with claim 2, **characterised in that** the cutting means (15, 15') are of elongate design.

4. Trocar in accordance with claim 3, **characterised in that** the cutting means are wires (15), blades or knives (15') connected to the connections (12, 13).

5. Trocar in accordance with any one of the preceding claims, **characterised in that** the cutting means (15, 15') project in arcuate-like manner or in pointed manner from the front end of the cutting bar (14).

6. Trocar in accordance with any of claims 3 to 5, **characterised in that** the cutting means (15, 15') span the front cross-section of the cutting bar (14) diametrically, or crosswise, or in star-like manner

7. Trocar in accordance with claim 6, **characterised in that** the cutting means (15, 15') span the free inner cross-section of the tubular cutting bar (14).

8. Trocar in accordance with any one of the preceding claims, **characterised in that** the transparent component (18) is of dome-like shape.

9. Trocar in accordance with any one of the preceding claims, **characterised in that** the cutting means (15, 15') have a spacing from the transparent component (18)

10. Trocar in accordance with claim 9, **characterised in that** the space is maintained by spacers (19).

## Revendications

1. Trocart pour opérations laparoscopiques de patients, comportant un tube (11) dans lequel une tige coupante (14) tubulaire est montée à déplacement axial et peut être immobilisée dans une position de coupe, à l'extrémité antérieure de laquelle sont agencés des raccords à haute fréquence (12, 13) susceptibles d'être connectés à un générateur de haute fréquence et des moyens de coupe (15, 15') électro-chirurgicaux, connectés à ces raccords, susceptibles d'être chauffés par le courant à haute fréquence et destinés à la coupe électro-chirurgicale de tissus lorsqu'on fait pénétrer le tube (11) dans le corps du patient, lesquels se trouvent dans la région de l'extrémité antérieure du tube (11) en position de coupe, un endoscope (17) étant logé dans la tige de coupe (14) avec son extrémité d'observation (17') antérieure directement derrière les moyens de coupe (15, 15'), **caractérisé en ce qu'**il est prévu entre la paroi intérieure de la tige de coupe (14) tubulaire et la paroi extérieure de l'endoscope (17) un espace intermédiaire formant une partie de l'espace intérieur (21) de la tige de coupe (14) tubulaire recevant l'endoscope (17) et susceptible d'être raccordé à un système d'alimentation (22) de gaz de rinçage ou de liquide de rinçage et/ou, dans la mesure où l'extrémité antérieure de la tige de coupe (14) tubulaire est fermée par un composant (18) transparent, il est prévu entre la paroi intérieure du tube (11) et la paroi extérieure de la tige de coupe (14) tubulaire un espace intermédiaire (20) susceptible d'être raccordé à un système d'alimentation (22) de gaz de rinçage ou de liquide de rinçage.

2. Trocart selon la revendication 1, **caractérisé en ce que** les moyens de coupe (15, 15') enjambent l'extrémité antérieure de la tige coupante (14).

3. Trocart selon la revendication 2, **caractérisé en ce que** les moyens de coupe (15, 15') sont réalisés oblongs.

4. Trocart selon la revendication 3, **caractérisé en ce que** les moyens de coupe sont des fils (15), des tranchants ou des lames (15') raccordés aux raccords (12, 13).

5. Trocart selon l'une des revendications précédentes, **caractérisé en ce que** les moyens de coupe (15, 15') font saillie en forme d'arc ou en pointe depuis l'extrémité antérieure de la tige de coupe (14).

6. Trocart selon l'une des revendications 3 à 5, **caractérisé en ce que** les moyens de coupe (15, 15') enjambent la section transversale antérieure de la tige de coupe (14) diamétralement ou en forme de croix.

7. Trocart selon la revendication 6, **caractérisé en ce que** les moyens de coupe (15, 15') enjambent la section transversale intérieure de la tige de coupe (14) tubulaire.

8. Trocart selon l'une des revendications précédentes, **caractérisé en ce que** le composant (18) transparent est réalisé en forme de coupole.

9. Trocart selon l'une des revendications précédentes, **caractérisé en ce que** les moyens de coupe (15, 15') présentent une distance par rapport au composant (18) transparent.

10. Trocart selon la revendication 9, **caractérisé en ce que** la distance est maintenue par des écarteurs (19).
